# EUROPEAN PATENT SPECIFICATION

(11) **EP 4 259 130 B1**
(45) Date of publication and mention of the grant of the patent: **04.03.2026**
(21) Application number: 21830531.6
(22) Date of filing: 10.12.2021
(51) Int. Cl.: A61K 31/415, A61K 39/00, A61K 45/06, A61K 31/165, A61K 31/5415, A61P 7/06

(54) **GARDOS CHANNEL INHIBITOR OR OTHER AGENTS FOR USE IN THE TREATMENT AND PREVENTION OF ANAEMIA OF INFLAMMATION**
GARDOS-KANALHEMMER ODER ANDERE MITTEL ZUR VERWENDUNG BEI DER BEHANDLUNG UND PRÄVENTION VON ANÄMIE BEI CHRONISCHER ERKRANKUNG
INHIBITEUR DU CANAL GARDOS OU AUTRES AGENTS POUR UNE UTILISATION DANS LE TRAITEMENT ET LA PRÉVENTION DE L'ANÉMIE INFLAMMATOIRE

(30) Priority: 11.12.2020 EP 20213523
(43) Date of publication of application: 18.10.2023
(73) Proprietor: Sanquin IP B.V., 1066 CX Amsterdam (NL)
(72) Inventor: VAN BRUGGEN, Robin, 1066 CX Amsterdam (NL); KLEI, Thomas Robert Leon, 1066 CX Amsterdam (NL)
(74) Representative: V.O.
(86) International application number: PCT/NL2021/050756
(87) International publication number: WO 2022/124900

(56) References cited:
- WO-A2-2006/116688
- C BRUGNARA ET AL: "Therapy with oral clotrimazole induces inhibition of the Gardos channel and reduction of erythrocyte dehydration in patients with sickle cell disease.", JOURNAL OF CLINICAL INVESTIGATION, vol. 97, no. 5, 1 March 1996 (1996-03-01), pages 1227 - 1234, XP055151940, ISSN: 0021-9738, DOI: 10.1172/JCI118537
- CAULIER A. ET AL: "Primary red cell hydration disorders: Pathogenesis and diagnosis", INTERNATIONAL JOURNAL OF LABORATORY HEMATOLOGY, vol. 40, no. Suppl. 1, May 2018 (2018-05-01), GB; US, pages 68 - 73, XP055806727, ISSN: 1751-5521, DOI: 10.1111/ijlh.12820
- DURPÈS MARIE-CLAUDE ET AL: "Effect of interleukin-8 and RANTES on the Gardos channel activity in sickle human red blood cells: Role of the Duffy antigen receptor for chemokines", BLOOD CELLS, MOLECULES & DISEASES, vol. 44, no. 4, April 2010 (2010-04-01), US, pages 219 - 223, XP055806811, ISSN: 1079-9796, DOI: 10.1016/j.bcmd.2010.02.001
- CONRAN NICOLA ET AL: "Inflammation in sickle cell disease", CLINICAL HEMORHEOLOGY AND MICROCIRCULATION, vol. 68, no. 2-3, 28 March 2018 (2018-03-28), NL, pages 263 - 299, XP055899461, ISSN: 1386-0291, DOI: 10.3233/CH-189012
- GANZ TOMAS: "Anemia of Inflammation", NEW ENGLAND JOURNAL OF MEDICINE, vol. 381, no. 12, 19 September 2019 (2019-09-19), pages 1148 - 1157, XP009527670, ISSN: 0028-4793
- TELEN M J: "RED BLOOD CELL SURFACE ADHESION MOLECULES: THEIR POSSIBLE ROLES IN NORMAL HUMAN PHYSIOLOGY AND DISEASE", SEMINARS IN HEMATOLOGY, W.B. SAUNDERS CO, US, vol. 37, no. 2, 1 April 2000 (2000-04-01), pages 130 - 142, XP008061588, ISSN: 0037-1963, DOI: 10.1016/S0037-1963(00)90038-6
- KLEI THOMAS R. L. ET AL: "The Gardos effect drives erythrocyte senescence and leads to Lu/BCAM and CD44 adhesion molecule activation", BLOOD ADVANCES, vol. 4, no. 24, 22 December 2020 (2020-12-22), pages 6218 - 6229, XP055806809, ISSN: 2473-9529, DOI: 10.1182/bloodadvances.2020003077

## Description

### Field of the invention

The invention relates to the field of therapy and prevention, specifically for anaemia of inflammation.

### Background of the invention

Anaemia of inflammation (Al) or anaemia of chronic disease affects a large number of patients that suffer from a range of diseases including autoimmune disorders, infectious diseases and cancer. AI is characterized by exacerbated red blood cell (RBC) breakdown and decreased erythropoiesis as a consequence of systemic inflammation. A multitude of inflammatory diseases such as bacterial and viral infections, including sepsis, but also autoimmune disorders and cancer, can result in AI. Currently, AI is mainly treated by transfusing red blood cells (RBC). Although transfusions restore haemoglobin levels, their efficacy is compromised by the increased degradation of all circulating RBC, including those transfused, making this group of patients one of the biggest consumers of blood products. Therefore, it would improve patient care as well as transfusion practice enormously if the increased degradation of RBC as well as the inhibition of erythropoiesis in patients suffering from AI could be prevented. In view thereof, it is thus of key importance to develop strategies that lead to improved erythropoiesis and prevent RBC breakdown during AI.

Brugnara et al. (J Clin Invest. 1996 Mar 1;97(5):1227-34. doi: 10.1172/JCI118537) describes that administration of clotrimazole, an inhibitor of Gardos channel, causes a reduction of erythrocyte dehydration in subjects suffering from sickle cell disease.

Caulier et al. (Int J Lab Hematol. 2018 May;40 Suppl 1:68-73. doi: 10.1111/ijlh.12820) describes the use of a Gardos channel blocker to prevent erythrocyte dehydration in the diseases sickle-cell disease and dehydrated hereditary stomatocytosis (DHS).

Durpès et al. (Blood Cells Mol Dis. 2010 Apr 15;44(4):219-23. doi: 10.1016/j.bcmd.2010.02.001) describes a loss of intracellular K⁺ upon deoxygenation of DARC positive sickle erythrocytes in the presence of IL-8 and RANTES in sickle erythrocytes.

Ganz et al. (N Engl J Med. 2019 Sep 19;381(12):1148-1157. doi: 10.1056/NEJMra1804281) reviews anemia of inflammation and treatment options.

Hence, there remains a need in the art for novel therapies for AI, in particular therapies that do not suffer from the disadvantages associated with the currently used therapy of transfusing RBC.

### Summary of the invention

It is an object of the present invention to provide methods for treatment or prevention of AI that combine improved erythropoiesis with prevention of RBC breakdown during AI.

The invention therefore provides a compound selected from the group consisting of:
- an inhibitor of the Ca²⁺-activated potassium channel (Gardos channel),
- an inhibitor of interaction of one or more chemokines with Duffy antigen receptor for chemokines (DARC), wherein said inhibitor is an antibody or antigen-binding part thereof that specifically binds to DARC, and
- a compound that inhibits activation of adhesion molecules expressed on erythrocytes wherein said adhesion molecules are Lu/BCAM and/or CD44,
for use in a method for the treatment or prevention of anaemia of inflammation.

In a preferred embodiment, the subject treated in accordance with the invention is not suffering from sickle cell disease. In a further preferred embodiment, the subject is not suffering from hereditary spherocytosis or hereditary xerocytosis.

In a further aspect, the invention provides a compound selected from the group consisting of:
- an inhibitor of the Ca²⁺-activated potassium channel (Gardos channel), and
- an inhibitor of interaction of one or more chemokines with Duffy antigen receptor for chemokines (DARC), wherein said inhibitor is an antibody or antigen-binding part thereof that specifically binds to DARC,
for use in a method for reducing or preventing erythrocyte dehydration in a subject suffering from chronic inflammation, wherein the subject is suffering from a disorder selected from the group consisting of a viral, bacterial, parasitic or fungal infection, sepsis, cancer, an auto-immune disorder, rejection after organ transplantation, and chronic kidney disease.

Said auto-immune disorder may be rheumatoid arthritis, systemic lupus erythematosus, vasculitis, sarcoidosis or inflammatory bowel disease. It is further preferred that the subject is not suffering from sickle cell disease. In a further preferred embodiment, the subject is not suffering from any one of sickle cell disease, hereditary spherocytosis or hereditary xerocytosis.

### Detailed description

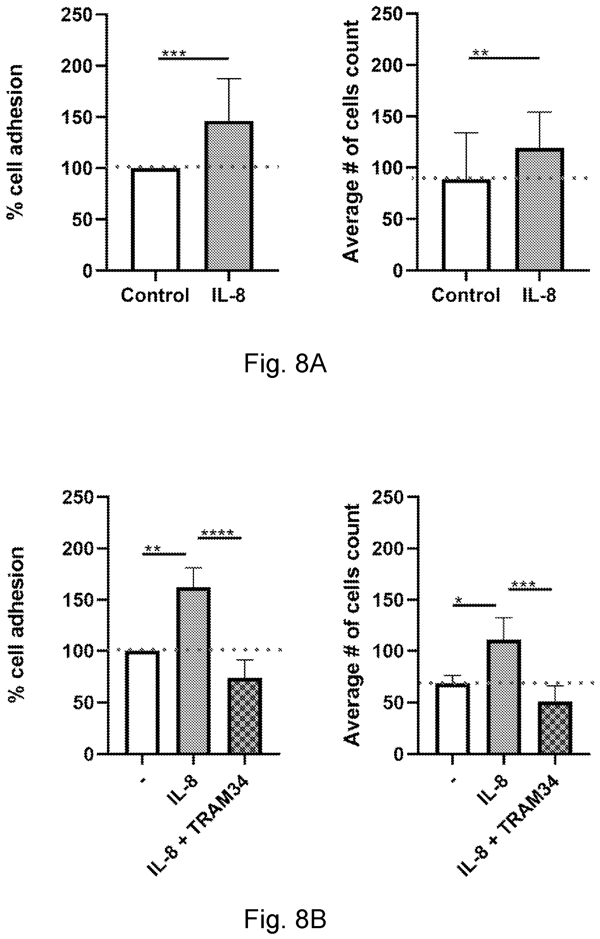

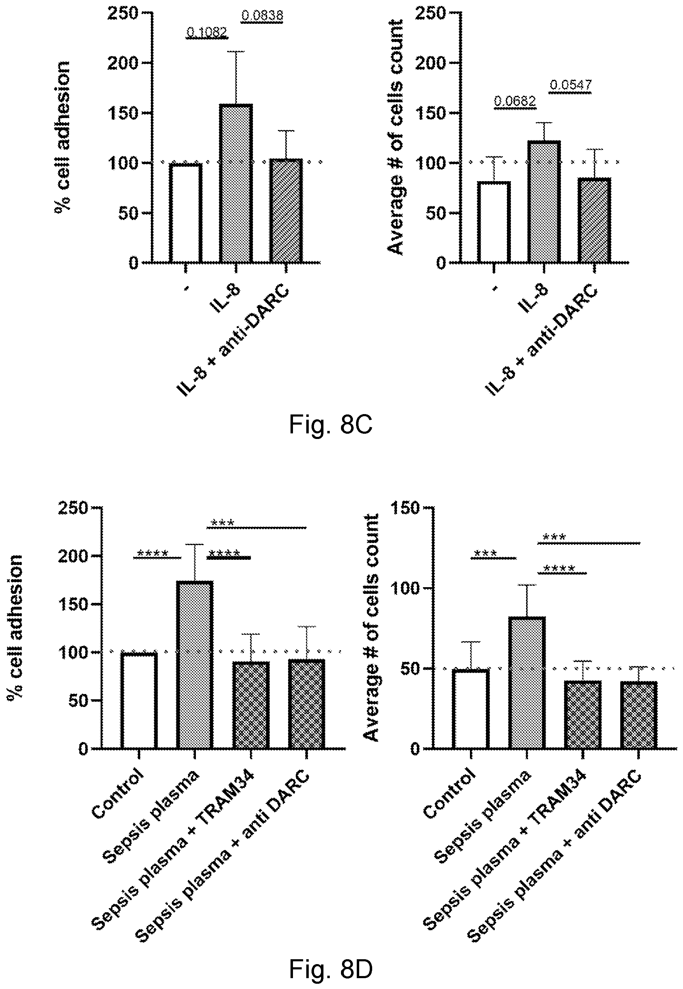

Degradation of RBCs in AI has always been regarded as the result of increased macrophage activation leading to the destruction of healthy RBCs. The present inventors found that binding of AI-related pro-inflammatory chemokines, such as IL-8, to the Duffy Antigen Receptor for Chemokines (DARC) on healthy RBCs induces an intrinsically regulated apoptotic-like process. Ultimately, this causes the RBCs to be targeted for phagocytosis by red pulp macrophages of the human spleen. The present inventors found that IL-8 induces Ca²⁺ influx in healthy erythrocytes. This leads to erythrocyte dehydration through K⁺ efflux, without a need for Ca²⁺ ionophores. In contrast to the literature cited above, these findings provide a physiological scientific basis for the prevention of chemokine-mediated erythrocyte dehydration in patients that suffer from anaemia of inflammation. In this disease, otherwise healthy erythrocytes are continuously exposed to high levels of chemokines induced by the inflammatory status. As erythrocyte dehydration, both in health and in disease, is associated with their destruction, preventing erythrocyte dehydration in response to chemokines may serve to prevent anaemia in anaemia of inflammation. Moreover, the inventors have established that DARC is expressed on erythroblasts and believe that pro-inflammatory chemokines inhibit erythroblast proliferation and/or differentiation via DARC-mediated signalling. Hence, binding of pro-inflammatory chemokines to DARC not only induces RBC degradation but also has an impact on erythropoiesis. Finally, the present inventors have shown that upon stimulation of IL-8, which binds to DARC, healthy erythrocytes show an increased adhesion to laminin-α5, which results from activation of its ligand Lu/BCAM on the surface of the RBCs. As previously demonstrated (Klei et al. 2020, Blood), the interaction between laminin-α5 and Lu/BCAM, following Ca2+ influx induced dehydration, induces hemolysis and subsequently degradation of RBCs. The increased adhesion of RBCs to laminin-α5 was counteracted by addition of an inhibitor of the Gardos channel (TRAM34). As such, adhesion to laminin-α5 is a direct measure for hemolysis mediated by the Gardos effect. Importantly, RBC that were incubated with serum of sepsis patients, containing pro-inflammatory cytokines and chemokines, also increase activation of Lu/BCAM and adhesion to laminin-α5, which could also be inhibited by TRAM34 (Figure 8).

Currently it is proposed that RBC retention in the spleen is mainly caused by a reduction of deformability due to dehydration. The present inventors found that RBC dehydration, as occurring during RBC ageing, storage and in sickle cell disease results in activation of adhesion molecules which, together, contribute to RBC clearance from the circulation (Klei et al. 2020, Blood).

In anaemia of inflammation (AI), erythropoiesis is decreased and RBC destruction is exacerbated. The present inventors found that healthy RBCs are targeted for destruction upon binding of IL-8 to DARC, thereby contributing to the rapid decrease of circulating RBCs as observed in AI. Furthermore, it was not only shown that DARC is capable of simultaneously binding various chemokines, but also that this strongly affects the signalling response in RBCs, with a large impact on their deformability and degradation. Without being bound by theory, it is believed that the IL-8-dependent signalling response that is elicited in healthy RBCs similarly occurs in erythroblasts, such that chronic inflammation may substantially impact erythropoiesis through DARC-mediated signalling. In summary, it is believed that DARC-mediated signalling contributes to increased breakdown of RBC as well as to the inhibition of erythropoiesis in AI.

In view of the above, the present inventors established that inhibition of the Gardos effect in otherwise healthy erythrocytes in AI offers an attractive treatment strategy, counteracting multiple deleterious mechanisms occurring during AI, in particular counteracting activation of adhesion molecules resulting in increased breakdown of RBC as well as to the inhibition of erythropoiesis.

Hence, the invention provides a compound selected from the group consisting of:
- an inhibitor of the Ca²⁺-activated potassium channel (Gardos channel),
- an inhibitor of interaction of one or more chemokines with Duffy antigen receptor for chemokines (DARC), and
- a compound that inhibits activation of adhesion molecules expressed on erythrocytes,
for use in a method for the treatment or prevention of anaemia of inflammation.

Also provided is a compound selected from the group consisting of:
- an inhibitor of the Ca²⁺-activated potassium channel (Gardos channel), and
- an inhibitor of interaction of one or more chemokines with Duffy antigen receptor for chemokines (DARC),
for use in a method for reducing or preventing erythrocyte dehydration in a subject suffering from chronic inflammation and/or chronic disease.

As used herein, the term "subject" encompasses humans and animals, preferably mammals. Preferably, a subject is a mammal, more preferably a human.

The term "therapeutically effective amount," as used herein, refers to an amount of a compound being administered sufficient to relieve one or more of the symptoms of the disease or condition being treated to some extent. This can be a reduction or alleviation of symptoms, reduction or alleviation of causes of the disease or condition or any other desired therapeutic effect.

As used herein, the term "prevention" refers to preventing or delaying the onset of a disease or condition, e.g. anaemia of inflammation or erythrocyte dehydration, and/or the appearance of clinical symptoms of the disease or condition in a subject that does not yet experience clinical symptoms of the disease. The term "treatment" refers to inhibiting the disease or condition, e.g. anaemia of inflammation, i.e., halting or reducing its development or at least one clinical symptom of the disease or condition, and/or to relieving symptoms of the disease or condition.

As used herein "reduced" means that the indicated activity (e.g. erythrocyte dehydration) is reduced by at least about 10%, preferably at least about 15%, more preferably at least about 20%, more preferably at least about 25%, more preferably at least about 50%, such as at least 60%, at least 70%, at least 80% or at least 90%, as compared to prior to administration of a compound used in accordance with the invention.

As used herein anaemia of inflammation (AI) , also referred to as anaemia of chronic disease (ACD), refers to a type of anaemia that affects subjects suffering from a condition that causes inflammation. In particular, AI refers to anaemia that affects subjects suffering from a condition that is associated with systemic inflammation.

As used herein "anaemia" refers to a decrease in the total amount of RBCs or haemoglobin in the blood, or a decreased ability of the blood to carry oxygen. Symptoms of anaemia include tiredness, weakness, shortness of breath, headache, confusion, and loss of consciousness. Severe anaemia can be life threatening. In one preferred embodiment, the anaemia is hemolytic anaemia. The term "hemolytic anaemia" as used herein refers to a decrease in the total amount of RBCs in a subject.

The anaemia of inflammation may be induced by an inflammatory disease, such as a viral, bacterial, parasitic or fungal infection, sepsis, cancer, an auto-immune disorder, rejection after organ transplantation, or chronic kidney disease and inflammation. Examples of the autoimmune diseases according to the present invention include, but are not limited to, arthritic diseases such as rheumatoid arthritis, juvenile idiopathic arthritis and psoriatic arthritis, inflammatory bowel diseases such as ulcerative colitis and Crohn's disease, systemic lupus erythematosus, scleroderma, multiple sclerosis, Behcet's disease, Sjogren's syndrome, chronic hepatitis and glomerulonephritis. In the case of cancer, AI may be caused by the cancer itself or by cancer treatment such as chemotherapy. Examples of cancers associated with AI include, but are not limited to, cancers like leukaemia, lymphoma, and myeloma and gastrointestinal, urinary tract, male genital, head and neck, and cervical and vaginal cancers.

Hence, in a preferred embodiment, the subject is suffering from a chronic disease and/or from chronic inflammation. As used herein "chronic" refers to a persistent or lasting disease or medical condition, such as for at least 6 months, preferably at least 1 year. In a preferred embodiment, said chronic inflammation and/or chronic disease is an inflammatory disease, such as bacterial or viral infection, an autoimmune diseases cancer or chronic kidney disease.

In a further preferred embodiment, the subject is suffering from a disorder selected from the group consisting of an inflammatory disease, such as a viral, bacterial, parasitic or fungal infection, sepsis, cancer, an auto-immune disorder (such as rheumatoid arthritis, systemic lupus erythematosus, vasculitis, sarcoidosis and inflammatory bowel disease), rejection after organ transplantation, and chronic kidney disease.

In a further embodiment the subject is suffering from anaemia of inflammation.

In one embodiment the subject is suffering from anaemia of inflammation and suffering from a disorder selected from the group consisting of an inflammatory disease, such as a viral, bacterial, parasitic or fungal infection, sepsis, cancer, an auto-immune disorder (such as rheumatoid arthritis, systemic lupus erythematosus, vasculitis, sarcoidosis and inflammatory bowel disease), rejection after organ transplantation and chronic kidney disease.

In a preferred embodiment, the subject is not suffering from sickle cell disease. In a further preferred embodiment, the subject is not suffering from hereditary spherocytosis or hereditary xerocytosis.

As demonstrated in the examples and described herein above, the inhibition of the Gardos effect that is contemplated with the methods as described herein has several effects on erythrocytes. In particular, one or more of erythrocyte dehydration, loss of deformability of erythrocytes and activation of adhesion molecules expressed on erythrocytes are counteracted. Hence, the treatment or prevention preferably counteracts erythrocyte dehydration, loss of deformability of erythrocytes and/or activation of one or more adhesion molecules expressed on erythrocytes such as Lu/BCAM and CD44, preferably counteracts all of erythrocyte dehydration, loss of deformability of erythrocytes and activation of one or more adhesion molecules expressed on erythrocytes such as Lu/BCAM and CD44.

As used herein "counteract" means that the indicated effect is reduced or that progression thereof is halted or slowed down. For instance, counteracting erythrocyte dehydration means that dehydration is reduced or that progression of dehydration is halted or slowed down. As used herein "reduced" means that the indicated activity is reduced by at least about 10%, preferably at least about 15%, more preferably at least about 20%, more preferably at least about 25%, more preferably at least about 50%, such as at least 60%, at least 70%, at least 80% or at least 90%, as compared to prior to administration of a compound used in accordance with the invention. As used herein "progression is halted" means that the relevant effect is maintained at approximately the same level as compared to prior to administration of a compound used in accordance with the invention. As used herein "progression is slowed down" means that the relevant effect is still increasing, but to a lower extent as compared to prior to administration of a compound used in accordance with the invention.

As used herein "deformability of erythrocytes" refers to the ability of erythrocytes to change shape, without hemolysing. Deformability is essential for successful passage through capillaries and splenic sinuses. "Loss of deformability of erythrocytes" refers to a decreased ability of an erythrocyte to deform, i.e. to change its shape. Deformability of erythrocytes and whether or not a compound used in accordance with the invention counteracts or inhibits loss of deformability can be measured using any suitable method known in the art, for instance using an automated rheoscope and cell analyser (ARCA) at a shear stress of 30 dyne/cm² (3 Pa) as described by Van Zwieten *et al.* in the presence and absence of the compound.

As used herein "erythrocyte dehydration" refers to cellular dehydration, in particular to leakage of Ca²⁺ into the cell, and efflux of K⁺ and H₂O. Dehydration typically results in an increase in mean cell haemoglobin concentration (MCHC) and an increase in density of the erythrocyte. Whether or not a compound is able to inhibit dehydration of erythrocytes can be determined by any method known in the art. For instance, potassium efflux from erythrocytes can be determined as a measure for erythrocyte dehydration. For instance using cellular assays wherein a detectable form or analog of potassium, such as ⁸⁶Rb is measured. For example, erythrocytes are exposed to ⁸⁶Rb and the uptake thereof in the presence and absence of the compound can be measured. Alternatively, K⁺ content in erythrocytes can be determined using ion selective electrodes.

Erythrocyte (RBC) dehydration is a characteristic feature of several hematologic disorders, including sickle cell anaemia, hereditary-xerocytosis (HX) and spherocytosis (HS), but has not previously been associated with healthy RBCs. Sickle cell anaemia, is characterized by red cell sickling, chronic hemolytic anaemia and occlusion of the microcirculation. In sickle cell disease a point mutation in the β-globin subunit of haemoglobin results in what is known as sickle hemoglobin (HbS). At low oxygen tension, HbS polymerizes and forms fibrous precipitates which can cause the onset of vaso-occlusive crises. Dehydrated sickle erythrocytes are even more prone to sickle, especially under conditions in which oxygen tension is also low. Activation of the Ca²⁺activated K⁺ efflux channel (Gardos channel) is the main cause of sickle erythrocyte dehydration. Thus, blocking of the Gardos channel has been proposed as a potential therapeutic strategy for preventing vaso-occlusive crises in sickle cell disease (e.g. Rivera *et al.* 2020). However, Rivera *et al.* measures Ca ionophore dependent K⁺ influx, instead of efflux. K⁺ influx is not regulated through the Gardos channel and is not a cause of erythrocyte dehydration. Moreover, Ataga *et al.* that showed that treatment of sickle cell patients with the Gardos channel inhibitor Senicapoc did not result in reduction of vaso-occlusive events. Also in sickle erythrocytes, a loss of intracellular K⁺ upon deoxygenation of DARC positive sickle erythrocytes in the presence of IL-8 and RANTES has been described (Durpès *et al.* 2010).

In a preferred embodiment, the compound used in accordance with the invention is an inhibitor of the Ca²⁺-activated potassium channel, also referred to as the Gardos channel. The Gardos channel is responsible for Ca²⁺-dependent K⁺ efflux from human erythrocytes, which is therefore known as the Gardos effect. An "inhibitor of the Gardos channel" as used herein refers to a compound that is able to inhibit potassium efflux from erythrocytes via this channel, preferably in anaemia of inflammation. "Inhibit" as used herein preferably means that the potassium efflux is reduced such that erythrocyte dehydration is reduced. Preferably potassium efflux is reduced by at least about 10%, preferably at least about 15%, more preferably at least about 20%, more preferably at least about 25%, more preferably at least about 50%, more preferably at least about 75%, more preferably at least about 80%, more preferably at least about 85%, more preferably at least about 90%, most preferably at least about 95%. In one embodiment, potassium efflux via the Gardos channel is essentially blocked or blocked.

Any Gardos channel inhibitor is suitable for use in accordance with the invention. Many of such inhibitors are currently known in the art, which are all suitable for use in the present invention. Non-limiting examples of Gardos channel inhibitors are charybdotoxin, imidazole and triazole derivatives such as clotrimazole (CLT) and analogs such as TRAM-34 (1-[(2-chlorophenyl)diphenylmethyl]-1H-pyrazole), miconazole, econazole, butoconazole, oxiconazole and sulconazole, ICA-17043 (4-fluoro-α-(4-fluorophenyl)-a-phenyl-benzene acetamide, also known as senicapoc^{®}), NS6180 (4-[[3-(trifluoromethyl)phenyl]methyl]-2H-1,4-benzothiazin-3(4H)-one), 11-phenyl-dibenzazepine, diphenylindanone, sulfonamide, nifedipine, 4-phenyl-4H-pyran and cyclohexadienes and (bicyclic) cyclohexadiene lactone. Suitable Gardos channel inhibitors are further described in US6028103, US2007185209, US2009036538, US2010056637, US6288122, US5441957, US5273992, US7709533, EP0781128, WO 96/08242, WO 2005/003143, WO 97/34589, WO 00/50026, WO 99/24034, US7119112, WO2004/016221, US20030134842, WO99/026628, US20020119953, US20090076157, US2009186810 and Wulff and Castle (2012). Yet another example of a Gardos channel inhibitor is an antibody or antigen-binding part thereof that blocks potassium efflux via the Gardos channel.

In a preferred embodiment, the Gardos channel inhibitor is selected from the group consisting of clotrimazole, TRAM-34, Senicapoc, NS6180, more preferably from the group consisting of clotrimazole, TRAM-34 and Senicapoc, more preferably the inhibitor is TRAM-34 or Senicapoc.

In another preferred embodiment, the compound used in accordance with the invention is an inhibitor of interaction of one or more chemokines with Duffy antigen receptor for chemokines (DARC), preferably on erythrocytes. Such inhibitor is herein also referred to as a "DARC inhibitor". Preferably at least binding of one or more chemokines selected from the group consisting of interleukin-8 (IL-8, CXCL8), RANTES (CCL5), MCP-1 (CCL2), CXCL5, CXCL6, CXCL8, CXCL11, CCL17, CXCL1, CXCL2, CXCL3, CXCL4, CCL7, CCL11, CCL13, CCL14, CCL1, CCL8, CCL16, CCL18, CXCL9, CXCL10 and CXCL13 is inhibited. Most preferably interaction of at least one of IL-8 and RANTES with DARC is inhibited, most preferably both IL-8 and RANTES. Interaction is preferably inhibited such that one or more processes resulting in potassium efflux via the Gardos channel is inhibited.

An "inhibitor of interaction of one or more chemokines with DARC" as used herein refers to a compound that is able to inhibit binding of one or more chemokines to DARC on erythrocytes, preferably in anaemia of inflammation. "Inhibit" as used herein preferably means that the binding is reduced such that erythrocyte dehydration is reduced. Preferably binding of one or more chemokines, more preferably efflux of potassium, is reduced by at least about 10%, preferably at least about 15%, more preferably at least about 20%, more preferably at least about 25%, more preferably at least about 50%, more preferably at least about 75%, more preferably at least about 80%, more preferably at least about 85%, more preferably at least about 90%, most preferably at least about 95%. In one embodiment, binding of one or more chemokines to DARC is essentially blocked or blocked. In a further preferred embodiment, potassium efflux via the Gardos channel is essentially blocked or blocked.

The inhibitor of interaction of one or more chemokines with DARC is an antibody or antigen-binding part thereof that specifically binds to DARC. The terms "specifically binds" and "specific for" as used herein refer to the interaction between an antibody, or antigen-binding part thereof, and its epitope. The terms mean that said antibody, or part thereof, preferentially binds to said epitope over other amino acid sequences or portions of the antigen or over other antigens. Although the antibody or part may non-specifically bind to other portions, amino acid sequences or antigens, the binding affinity of said antibody or part for its epitope is significantly higher than the non-specific binding affinity of said antibody or part for other portions, amino acid sequences or antigens. Preferably, the antibody or part thereof is a blocking antibody or part thereof. In one embodiment, the antibody of part thereof binds to the DARC Fy6 epitope, which is the epitope bound by chemokines such as IL-8. Preferably, the antibodies or parts thereof are human or humanized antibodies or parts thereof. Any anti-DARC antibody or anti-Fy6 antibody, including human or humanized murine antibodies, that inhibits binding of chemokines to DARC known in the art can be used in accordance with the invention. An exemplary DARC antibody for use in the present invention is anti-DARC antibody Fy6, an antibody as described in EP1877030 or as described by Patterson *et al.,* (2002), anti-Fya antibodies, anti-Fyb antibodies and/or anti-Fy3 antibodies. These antibodies are preferably human or humanized antibodies. Suitable anti-DARC antibodies for use in the present invention can for instance be derived from the blood of donors, preferably are isolated from donor plasma. Hence, in a preferred embodiment, the inhibitor of interaction of one or more chemokines with DARC is selected from the group consisting of anti-Fya antibody or antigen-binding part thereof, anti-Fyb antibody or antigen-binding part thereof, anti-Fy3 antibody or antigen-binding part thereof, anti-Fy6 antibody or antigen-binding part thereof and combinations thereof, more preferably selected from the group consisting of anti-Fya antibody, anti-Fyb antibody, anti-Fy3 antibody, anti-Fy6 antibody and combinations thereof. Such antibodies are commercially available, for instance from Sanquin (Amsterdam, The Netherlands).

In another preferred embodiment, the compound used in accordance with the invention is a compound that inhibits activation of adhesion molecules expressed on erythrocytes. As used herein "activation of adhesion molecules expressed on erythrocytes" refers to increase of activation of any adhesion molecule on the surface of erythrocytes. Non-limiting examples of such adhesions molecules are Lu/BCAM, CD44, CD47, CD147, LW/ICAM-4). In accordance with the invention, activation of at least Lu/BCAM and/or CD44 expressed on erythrocytes is counteracted. Activation of adhesion molecules and whether or not a compound inhibits such activation can be determined using any method known in the art. This is done by quantifying erythrocyte adherence to substrates such as laminin-α5 and hyaluronic acid, the respective ligands of Lu/BCAM and CD44, for instance as demonstrated in the examples herein. Hence, preferably "activation of adhesion molecules expressed on erythrocytes" refers to increased activation of Lu/BCAM and CD44 adhesion molecules as determined by increased adhesion of the erythrocytes to laminin-α5 and/or hyaluronic acid (HA).

A "compound that inhibits activation of adhesion molecules expressed on erythrocytes" as used herein refers to a compound that is able to inhibit activation of adhesion molecules such that degradation of erythrocytes in anaemia of inflammation is reduced. "Inhibit" as used herein thus preferably means that activation of adhesion molecules on erythrocytes is reduced such that erythrocyte degradation is reduced. Preferably activation of adhesion molecules on erythrocytes is reduced by at least about 10%, preferably at least about 15%, more preferably at least about 20%, more preferably at least about 25%, more preferably at least about 50%, more preferably at least about 75%, more preferably at least about 80%, more preferably at least about 85%, more preferably at least about 90%, most preferably at least about 95%. In one embodiment, activation of at least one adhesion molecule on erythrocytes is essentially blocked or blocked.

Any compound that inhibits activation of adhesion molecules Lu/BCAM and/or CD44 on erythrocytes is useful in the methods of the present invention. Suitable examples include antibodies against adhesion molecules, such as anti-Lu/BCAM, and/or anti-CD44 antibodies. A person skilled in the art is well capable of determining whether a compound inhibits activation of adhesion molecules on erythrocytes, for instance by measuring the frequency of adhesion of erythrocytes to ligands that are specifically recognized by said adhesion molecules. Suitable experiments for such measurement is a flow assay as described in the examples herein, wherein erythrocyte adhesion to e.g. laminin-α5 or hyaluronic acid in response to IL-8 or serum of sepsis patients is increased. Whether or not a compound is capable of inhibiting activation of adhesion molecules on erythrocytes can be assessed by determining whether or not such compound is capable of inhibiting the increased adhesion of erythrocytes in response to IL-8 or serum of sepsis patients.

A compound used in accordance with the invention is preferably administered in a pharmaceutical composition comprising the compound and at least one pharmaceutically acceptable carrier, diluent and/or excipient. By "pharmaceutically acceptable" it is meant that the carrier, diluent or excipient must be compatible with the other ingredients of the formulation and not deleterious to the recipient thereof. In general, any pharmaceutically suitable additive which does not interfere with the function of the active compounds can be used. A pharmaceutical composition used according to the invention is preferably suitable for human use.

Examples of suitable carriers comprise a solution, lactose, starch, cellulose derivatives and the like, or mixtures thereof. In a preferred embodiment said suitable carrier is a solution, for example saline. For making dosage units, e.g. tablets, the use of conventional additives such as fillers, colorants, polymeric binders and the like, is contemplated. Examples of excipients which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain, in addition to materials of the above type, a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor. Compositions for intravenous administration may for example be solutions of the compounds of the invention in sterile isotonic aqueous buffer. Where necessary, the intravenous compositions may include for instance solubilizing agents, stabilizing agents and/or a local anesthetic to ease the pain at the site of the injection.

Suitable routes for administration of the compounds is within the capabilities of a person skilled in the art. Compounds used in accordance with the invention can be administered to a subject by a variety of routes. For example, the compound can be administered by any suitable parenteral or nonparenteral route, including, for example, topically (e.g., cream, ointment,eyedrops), or nasally (e.g., solution, suspension). Parenteral administration can include, for example, intraarticular, intramuscular, intravenous, intraventricular, intraarterial, intrathecal, subcutaneous, or intraperitoneal administration. Further, the compound may be administered to a subject in hospital via infusion or via injection from a healthcare professional. In particular, small molecules can be administered via oral or parenteral routes. Proteinaceous molecules, including antibodies and parts thereof, may also be administered via oral or parenteral routes, but are preferably administered by injection or infusion, preferably intravenous injection or infusion.

The exact dose and regimen of these compounds and compositions thereof will be dependent on the biological activity of the compound per se, the age, weight and sex of the subject, the needs of the individual subject to whom the medicament is administered, the degree of affliction or need and the judgment of the medical practitioner. In general, parenteral administration requires lower dosages than other methods of administration which are more dependent upon adsorption. However, the dosages for humans are preferably 0.001 - 10 mg per kg body weight. In general, enteral and parenteral dosages will be in the range of 0.1 to 1.000 mg per day of total active ingredients.

As described herein above, current treatment of anaemia of inflammation comprises transfusion with either whole blood or an erythrocyte containing fraction thereof to compensate for the loss of erythrocytes. The efficacy of transfusion is however, compromised by the increased degradation of transfused erythrocytes, in addition to degradation of endogenous erythrocytes. Now that the present inventors have identified possibilities to counteract erythrocyte dehydration and degradation in anaemia of inflammation it has also become possible to counteract dehydration and/or degradation of transfused erythrocytes by combining both treatments. Hence, in one embodiment of the invention the treatment or prevention of anaemia of inflammation with a compound in accordance with the invention is combined with erythrocyte transfusion. This can be any type of transfusion whereby a patient is transfused with erythrocytes, such as whole blood transfusion or transfusion with a blood fraction containing or comprising erythrocytes. This way both dehydration and degradation of endogenous erythrocytes and of transfused erythrocytes is counteracted. Such combination therapy is particularly advantageous in the treatment of anaemia of inflammation because in that case erythrocyte counts have typically already dropped in the subject suffering from anaemia of inflammation.

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

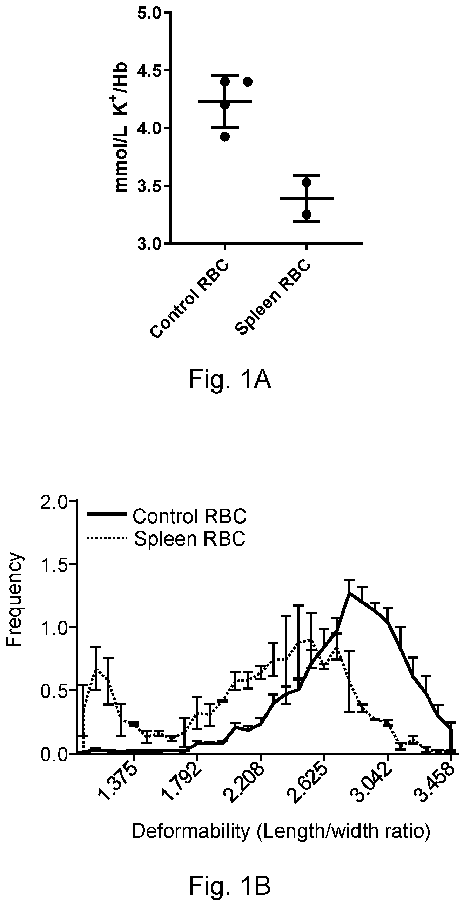

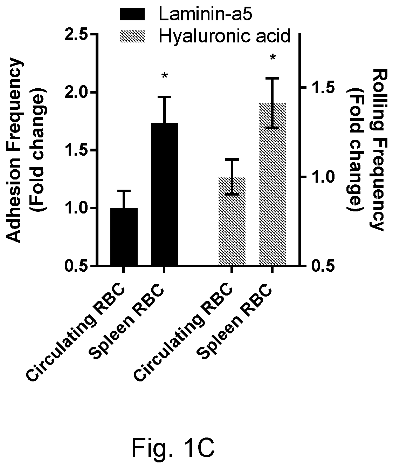

Figure 1: **Senescent RBC in the spleen are less deformable and express activated adhesion molecules**
   (A) Human spleen tissue was treated with collagenase buffer to create a single cell suspension from which RBC were isolated. RBC from human spleen and from the circulation were lysed and intracellular potassium was measured by ion-specific electrodes (spleen RBC n=2). (B) An automated rheoscope and cell analyzer was used to measure deformability of RBC from circulation and from spleen. RBC were subject to a shear stress of 10dyn/cm² after which the ability of RBCs to deform (length over width ratio) was automatically measured. Black barred line indicates deformability of 5 healthy controls, grey indicates deformability of RBC from 2 spleen samples. (C) RBC from circulation and from spleen were flown over a hyaluronic acid or laminin-α5-coated chamber under a shear stress of 0.2dyn/cm². As RBC adhere firmly to laminin-α5 but instead roll on hyaluronic acid, these two parameters were quantified by EVOS microscopy and are a readout for adhesion molecule activation4,5 (N=4-5, T-test, *P<0.05).

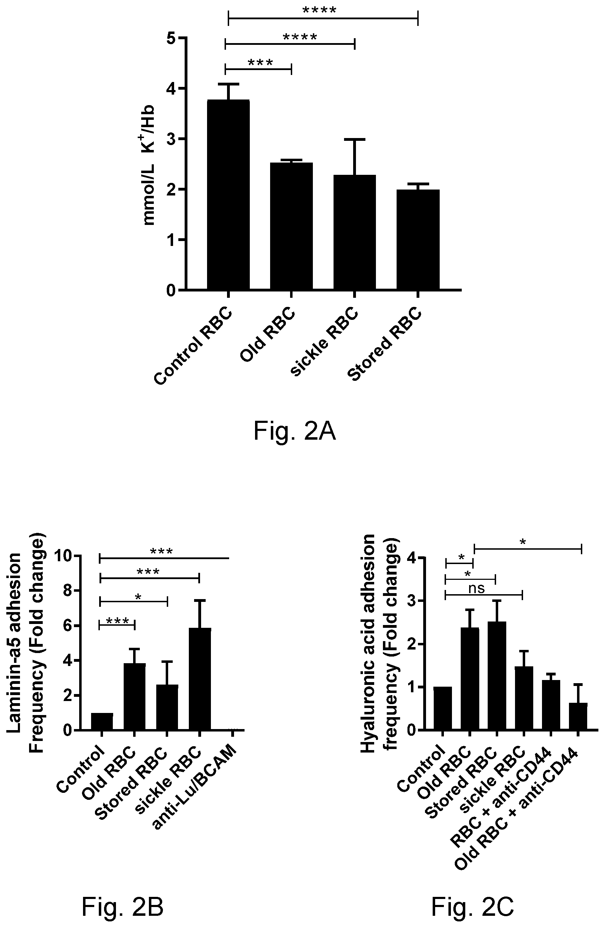

Figure 2: **RBC dehydration is associated with adhesion molecule activation**
   (A) RBCs were either isolated by density centrifugation to obtain old RBC, or from whole blood of sickle cell patients or isolated after storage for 4 weeks in the RBC storage medium Saline Adenine Glucose Mannitol (SAGM) after which intracellular potassium was measured by ion-specfic electrodes (n=3-8, one-way ANOVA, ; **P<0.01;***P<0.001). (B-C) A total of 1e7 control, old, stored and sickle RBC were flown over a laminin-α5 IBIDI chamber at 0.2dynes/cm² and adhesion frequency was assessed by microscopy. (n=3-7, one-way ANOVA, ±SEM). The same flow experiment was performed on hyaluronic acid. Here rolling frequency was quantified instead of adhesion frequency (n=3-7, T-test, ±SEM, *P<0.05; **P<0.01; ***P<0.001; ****P<0.0001).
Figure 3: A **murine model of anemia of inflammation**
   To induce AI, animals were injected intraperitoneally with 5 × 108 particles per mouse of heat-killed B abortus (strain 1119-3). Control mice were injected intraperitoneally with normal saline.
Figure 4: **IL-8 binding to RBC results in their dehydration and adhesion molecule activation**
   (A-B) A total of 1x106 RBC were incubated with 50nM of IL-8 for 30minutes at 37C. During this time span, Ca²⁺ influx (Fluo4) was measured by flow cytometry (n=4, T-test, *P<0.05) (C) The deformability of RBC exposed to 50nM of IL-8 was assessed by ARCA.
Figure 5: **Erythrocyte precursors bind SDF-1 in a DARC-dependent fashion**
   (A) A total of 1x106 RBC was incubated with 50nM of IL-8 for 30minutes at 37C. During this time span, Ca²⁺ influx (Fluo4) was measured by flow cytometry (n=4, T-test, *P<0.05) (B) The Fy6 DARC epitope was stained by monoclonal antibody and quantified by flow cytometry. Data is normalized to RBCs (n=6, one-way ANOVA, *P<0.05, **P<0.01).

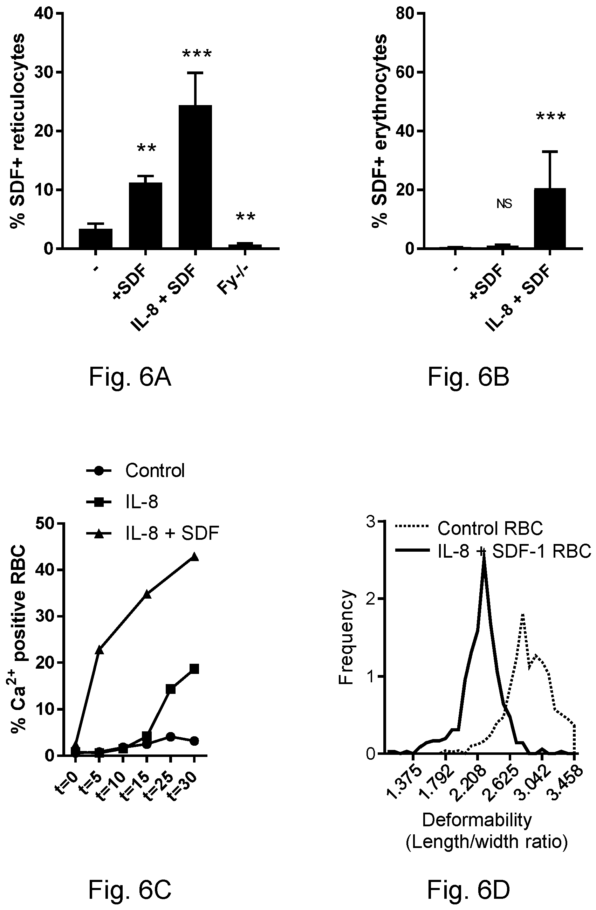

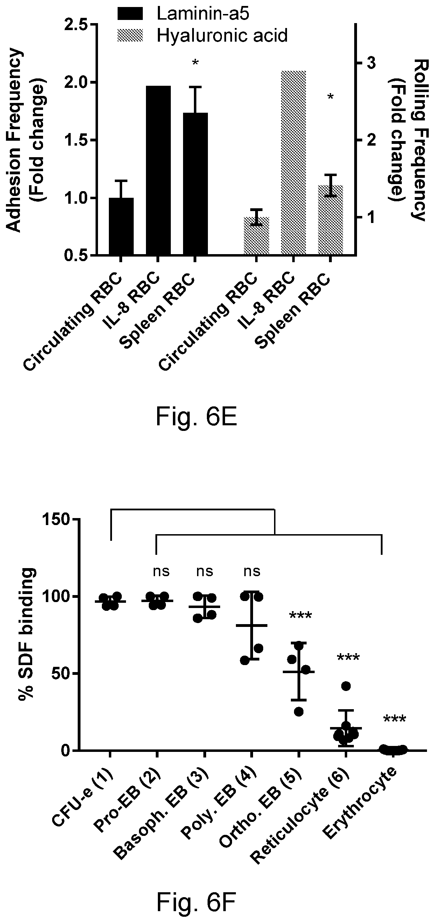

Figure 6: **IL-8 enhances SDF-1 binding to RBC which results in their dehydration**
   (A-B) SDF-1 was added to reticulocytes or RBC in the absence or presence of 50nM of IL-8. SDF-1 binding to reticulocytes was quantified by flow cytometry (N=4, one-way ANOVA, **P<0.01, ***P<0.001). (C) 50nM of IL-8 was added to RBC in the presence or absence of SDF-1 and Ca²⁺ influx was quantified by flow cytometry (Fluo-4). (D) Deformability of control and RBC treated with 50nM of IL-8 and 1µM of SDF-1 as assessed by ARCA. (E) A total of 1e7 control, IL-8-stimulated and spleen RBC were flown over a laminin-α5 and hyaluronic acid-coated IBIDI chambers. Adhesion and rolling frequency was quantified as previously described. (F) SDF-1 was added to cultured erythroblasts. The various stages of differentiation were identified based on CD71 and CD235a expression. SDF-1 binding was quantified by flow cytometry. (n=4, one-way ANOVA, ***P<0.001).

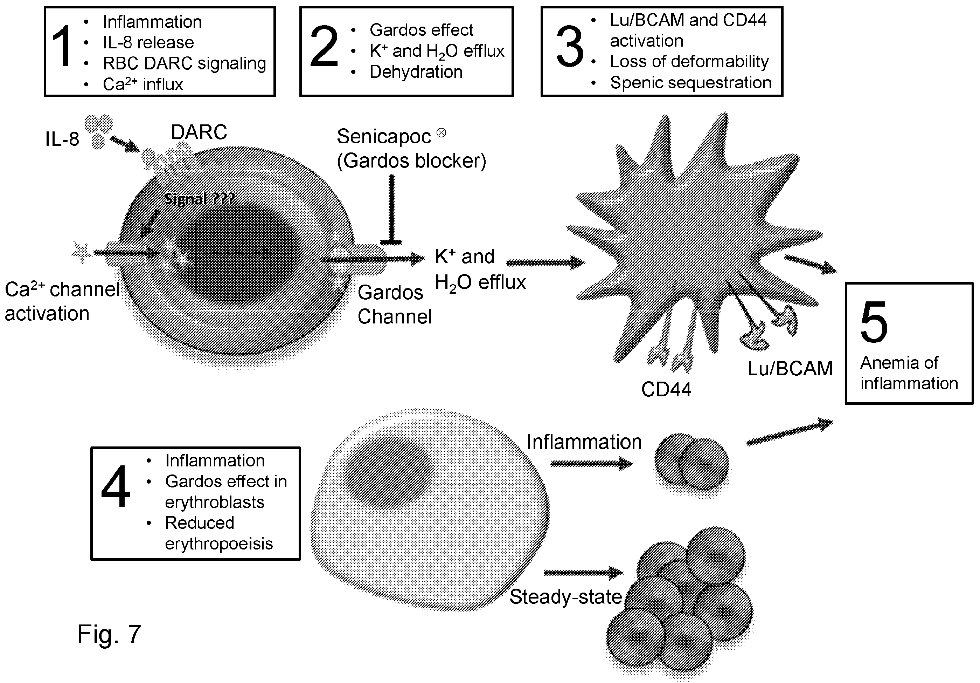

Figure 7: **Model of the role of DARC in anemia of inflammation**
   Depicted are the hypothesized roles of DARC in the pathophysiology of anemia of inflammation, both on the level of RBC destruction as well as on the inhibition of erythropoiesis.
Figure 8. **Adhesion of donor RBC to laminin-α5 in response to IL-8 and sera from sepsis patients**
   (A) Donor RBCs were incubated with Il-8 for 30 min at 37°C prior to the adhesion assay. (B) Donor RBCs were first incubated with TRAM34 for 30 min, and then with IL-8 for 30 min at 37°C prior to the adhesion assay. (C) Donor RBCs were first incubated with anti-DARC antibodies for 30 min, and then with IL-8 for 30 min at 37°C prior to the adhesion assay. (D) Donor RBCs were first incubated with TRAM34 or anti-DARC antibodies for 30 min, and then with sera of sepsis patients for 30 min at 37°C prior to the adhesion assay.

### Examples

### Example 1

### Materials and methods

### Blood samples and isolation of dense and light erythrocytes

Heparinised venous blood was obtained from healthy volunteers after informed consent. Blood studies were approved by the Medical Ethical Committee of Sanquin Research and performed in accordance with the 2013 Declaration of Helsinki. Erythrocytes were isolated by centrifugation of whole blood at 240g for 15 min. Next, plasma and buffy coat were removed and erythrocytes were washed twice with saline-adenine-glucose-mannitol medium (SAGM medium, 150mM NaCl, 1.25mM adenine, 50mM glucose, 29mM mannitol, pH 5.6; Fresenius SE). Washed erythrocytes were then either used for experiments or stored in SAGM for up to 4 weeks. Dense and light erythrocytes were isolated using Percoll (GE Healthcare, Little Chalfont, UK) density centrifugation. Briefly, isotonic Percoll was prepared by adding 8.1ml 10x PBS per 100ml Percoll. Next, Percoll buffer (26.3g/L BSA, 132mM NaCl, 4.6mM KCl, 10mM HEPES) was used to dilute isotonic Percoll to 1.096g/mL (80%), 1.087g/mL (71%), 1.083 g/mL (67%) 1.080 g/mL (64%) and 1.060g/mL (40%). Percoll dilutions were stacked in a 15mL tube, 2mL of isolated erythrocytes were added on top and centrifuged at 2100g for 15min at RT. Erythrocytes isolated from the fraction denser than 1.096 g/mL Percoll were defined as dense and aged erythrocytes whereas erythrocytes lighter than 1.080g/mL Percoll are here defined as light and young erythrocytes.

### Flow cytometry and staining procedures

Flow cytometric analysis was performed on the LSRII + HTS (BD Biosciences, Franklin Lakes, US) and data were analysed by FACS Diva software (BD Biosciences, Franklin Lakes, US). Erythrocytes were stained with either 1 µM FLUO-4 (Invitrogen, Carlsbad, US) or with 0.1 µM PBFI (Invitrogen, Carlsbad, US) supplemented with 0.4% pluronic and stimulated with 500uM propranolol or 10uM Valinomycin (All Sigma-Aldrich, Spruce, US). Blocking experiments were performed using 25 µM BAPTA-AM, 10µM TRAM-34, 25 µM Calpain 1 inhibitor (A6185), 1µg/ml DFP (All Sigma-Aldrich, Spruce, US) or 40 µM ZVAD-FMK (R&D systems, Minneapolis, US). α2,3-linked sialic acid was quantified by flow cytometry using biotinylated Maackia Amurensis type II lectin (Vector Laboratories, Peterborough, UK) followed by streptavidin alexa fluor 488 conjugation (ThermoFisher, Waltham, US). Glycophorin-A and Glycophorin-C expression on erythrocytes was quantified by anti-Glycophorin-A-PE (M1732, Sanquin, Amsterdam, NL) and anti-GpC (BRIC10 and BRIC4, a kind gift from IBRGL, Bristol). For every flow cytometric experiment where we determine SDF-1 binding to erythrocytes and its precursors we used biotinylated SDF-1 antibody listed in supplementary table 1 followed by streptavidin-647 conjugation. Afterwards, we took along nuclear staining (hoechst), anti-transferrin receptor (anti-CD71-FITC) and glycophorin-A staining (anti-CD235a-PE) to distinguish between the various stages of erythroid development. In case we had to perform additional stainings, such as in the case of determining Fy epitope exposure (e.g. Fy^{a}, Fy^{b}, Fy³ or Fy⁶) on SDF-interacting and non-interacting reticulocytes, we switched the order to ensure antigen-specific staining. In short, we first stained for Fy epitopes followed by either secondary anti-human-405 (for Fy^{a} and Fy^{b}) or anti-mouse-405 (for Fy³ or Fy⁶) after which we stained for SDF followed by streptavidin-647 conjugation, after which we again took along anti-CD71-FITC and anti-235a-PE). To further ensure antigen-specific staining we took along the appropriate IgG isotype controls. Exogenous addition of SDF-1 to erythroid cells was performed at 37°C for 30minutes whereas staining was performed at 4°C.

### Erythrocyte deformability

Deformability of young and aged erythrocytes was measured using an automated rheoscope and cell analyser (ARCA) at a shear stress of 30 dyne/cm² (3 Pa) as described previously (van Zwieten *et al.*).

### Flow assays

Erythrocyte adhesion to laminin-α5 and hyaluronic acid was assessed by coating 0.5µg laminin-511 (BioLamina, Sundyberg, Sweden) or 7.5ug of hyaluronic acid (Sigma-Aldrich, Spruce, US) dilutes in HEPES buffer (132mM NaCl, 20mM HEPES, 6mM KCl, 1mM MgSO₄, 1.2mM K₂HPO₄, 1mM Ca²⁺ all from Sigma-Aldrich, Spruce, US) per lane through passive adsorption on an uncoated IBIDI u-slideVI^{0.4} or ibiTreat µ-slideVI^{0.4} flow chamber (IBIDI). Erythrocytes were flown over in HEPES⁺ medium (HEPES buffer as described above supplemented with 0.5% human serum albumin and 1mg/ml glucose) unless stated otherwise. Adhesion was quantified by EVOS microscopy (ThermoFisher, Waltham, US) and image analysis software Vision4D (Arivis, Rostock, Germany). Experimental data were analysed using Graphpad Prism 6 software. Data are presented as mean ±SD unless otherwise indicated in the figure legends. The data were assumed to follow a normal distribution.

### Erythroblast culturing

Erythroblasts and reticulocytes of mixed stages were cultured as described perviously (van den Akker *et al.;* Leberbauer *et al.,* Heideveld *et al.*)

*Adhesion of donor RBC to* laminin-α5 *in response to IL-8 and sera from sepsis patients.*

Erythrocyte adhesion to laminin-α5 and hyaluronic acid was assessed by coating 0.5µg laminin-511 (BioLamina, Sundyberg, Sweden) or 7.5ug of hyaluronic acid (Sigma-Aldrich, Spruce, US) dilutes in HEPES buffer (132mM NaCl, 20mM HEPES, 6mM KCl, 1mM MgSO4, 1.2mM K2HPO4, 1mM Ca2+ all from Sigma-Aldrich, Spruce, US) per lane through passive adsorption on an uncoated IBIDI u-slideVI0.4 or ibiTreat µ-slideVI0.4 flow chamber (IBIDI). Erythrocytes were flown over in HEPES+ medium (HEPES buffer as described above supplemented with 0.5% human serum albumin and 1mg/ml glucose) unless stated otherwise. Adhesion was quantified by EVOS microscopy (ThermoFisher, Waltham, US) and image analysis software Vision4D (Arivis, Rostock, Germany). Experimental data were analysed using Graphpad Prism 6 software. Data are presented as mean ±SD unless otherwise indicated in the figure legends. The data were assumed to follow a normal distribution.

Donor RBCs were incubated with Il-8 for 30 min at 37°C prior to the adhesion assay. For determining the effect of TRAM34, donor RBCs were first incubated with TRAM34 (Sigma-Aldrich, Inc) for 30 min, and then with IL-8 for 30 min at 37°C prior to the adhesion assay. For determining the effect of anti-DARC antibodies, donor RBCs were first incubated with anti-DARC antibodies (isolated from human donor plasma, commercially available from Sanquin, Amsterdam, The Netherlands) for 30 min, and then with IL-8 for 30 min at 37°C prior to the adhesion assay. For determining the effect of serum of sepsis patients, donor RBCs were first incubated with TRAM34 or anti-DARC antibodies for 30 min, and then with sera of sepsis patients for 30 min at 37°C prior to the adhesion assay.

### Results

Every day billions of senescent RBCs are degraded in the spleen during steady state RBC turnover. Red pulp macrophages of the spleen recognize and clear senescent RBCs and are equipped with the machinery to degrade RBCs and recycle iron for erythropoiesis. However, the exact mechanisms by which senescent RBCs are recognized, trapped and ultimately broken down remain largely unclear. Currently it is proposed that RBC retention in the spleen is mainly caused by a reduction of deformability due to dehydration. Within the spleen, RBCs need to traverse endothelial fenestrae in order to recirculate. Although healthy, deformable, RBCs can pass these fenestrae, non-deformable, aged, RBCs are trapped, allowing red pulp macrophages to recognize and phagocytose these cells. Next to loss of deformability due to dehydration, old RBCs activate adhesion molecules including Lu/BCAM and CD44 on their surface, which contributes to retention of aged RBCs in the spleen by binding to laminin-α5 and hyaluronic acid in this organ. Indeed, when studying RBCs isolated from the human spleen, it was found that there is a large subpopulation of dehydrated RBCs (Fig. 1a) that display decreased deformability (Fig. 1b). Furthermore, these RBCs activate adhesion molecules Lu/BCAM and CD44, which makes them adhere significantly more frequent to laminin-α5 and hyaluronic acid (Fig 1c). Thus, RBC that are destined to be broken down are characterized by dehydration, decreased deformability and adhesion molecule activation.

RBC loss of deformability due to dehydration is not only occurring during RBC aging but also during RBC storage for transfusion and in sickle cell disease (Fig. 2a) which are two situations in which exacerbated RBC clearance is observed. Strikingly, in these situations, adhesion molecule activation is also observed (Fig. 2b-c), strongly suggesting that under conditions of increased RBC breakdown, the same intrinsic changes in the RBC lead to their destruction as under normal physiological aging.

RBC dehydration and concomitant loss of deformability occurs when RBCs are incapable of maintaining intracellular homeostasis. This is characterized by transient leakage of Ca²⁺ into the cell, causing activation of the Ca²⁺-dependent K⁺ efflux channel known as the Gardos channel which is accompanied by H₂O efflux. This phenomenon is termed the Gardos effect and is considered to be a hallmark of RBC ageing. We previously found that the Gardos effect in aged RBCs directly causes loss of deformability as well as the activation of the RBC adhesion molecules Lu/BCAM and CD44 (Klei et al. 2020, Blood Advances) not only in stored but also aged an sickle erythrocytes. This indicates that RBC dehydration and adhesion molecule activation, which we found to contribute to retention of erythrocytes in the spleen, are intricately connected.

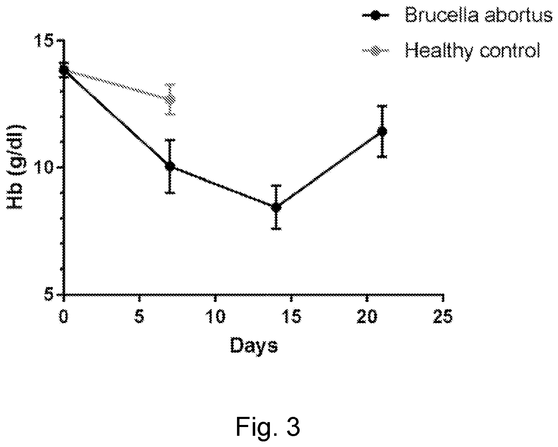

In anaemia of inflammation (AI), erythropoiesis is decreased and RBC destruction is exacerbated. This is illustrated for instance in patients on the ICU, who's hemoglobin levels are rapidly decreasing in time, without any active bleeding. Moreover, in a mouse model of AI, using heat-killed *B. abortus* to induce inflammation, the same impact on RBC destruction as well as erythropoiesis can be observed (Fig. 3).

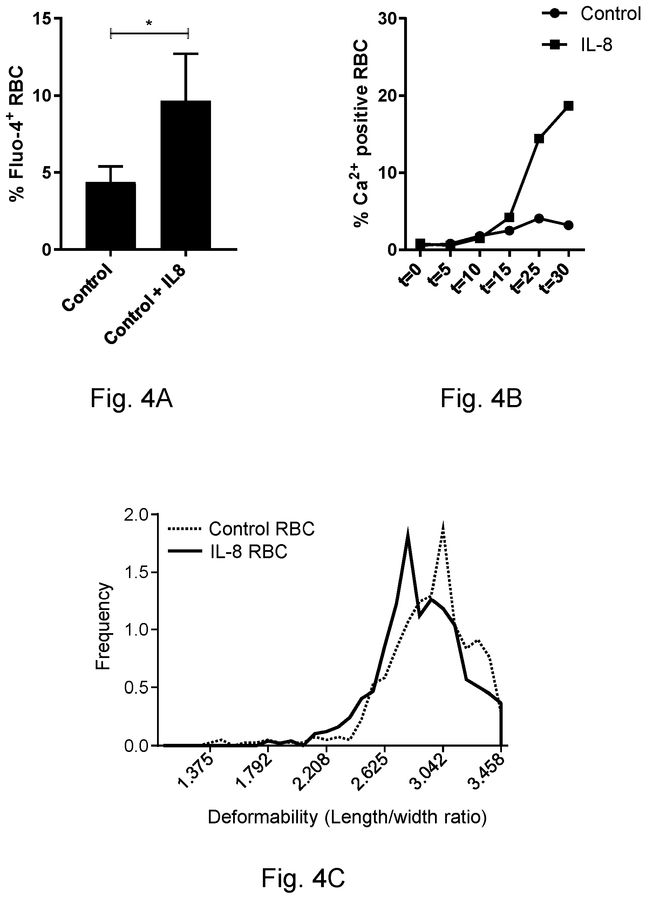

The increased RBC destruction in AI has been attributed to inflammation-mediated hyperactivation of splenic macrophages. However, using a highly sensitive flow cytometry-based assay we found that incubation of RBCs with IL-8 was sufficient to induce a transient DARC-dependent rise of intracellular calcium levels in a subset of RBCs (Fig. 4a-b). Although only a subset of RBCs responded to IL-8 treatment, this was found to have an effect on the deformability of the total population (Fig. 4c). These results show a direct effect of this pro-inflammatory chemokine on the integrity of RBCs, which may contribute to their degradation. The short-term effects of chemokine binding to RBCs, as presented here, may very well be an under-representation of how long-term chemokine binding to RBCs, as is the case in AI, may affect RBC hydration status (see figure 6).

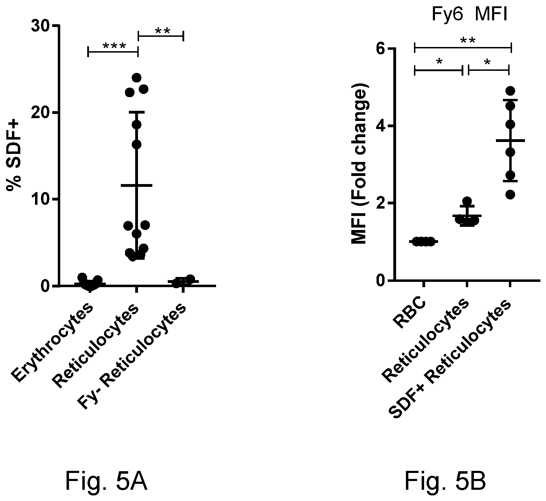

DARC has been suggested to be a non-signalling receptor, functioning merely as a chemokine sink on circulating RBCs. However, chemokine binding to DARC has not been studied in detail and many aspects of chemokine binding to DARC have yet to be revealed. This is underscored by a recent finding where we identified that SDF-1, the chemokine that restricts neutrophils to the bone marrow, was found to bind to DARC specifically on erythroid progenitors (Klei et al., 2019 Sci Rep.). We found that, in contrast to RBCs, erythroid progenitors bind SDF-1 in a DARC-dependent fashion (Fig. 5a). We established that DARC can be present on the RBC membrane in distinct conformations, as is reflected by a relatively higher accessibility/exposure of the so-called Fy6 epitope (Fig. 6b) on SDF-1-interacting reticulocytes (Fig. 5b).

As IL-8, similar to SDF-1, has been described to bind to the DARC Fy6 epitope, we initially aimed to block SDF-1 binding to DARC by IL-8. To our surprise, in the presence of IL-8, reticulocytes were binding significantly more SDF-1 instead of less (Fig. 6a). Even more striking was the finding that IL-8 allowed SDF-1 to bind to mature RBCs (Fig. 6b). As SDF-1 is a homeostatic chemokine we questioned whether the IL-8-induced binding of SDF-1 to DARC on RBC would dampen IL-8 associated RBC dehydration (figure 5). However, in the presence of SDF-1, IL-8 induced an even stronger Ca²⁺ influx (Fig. 6c) causing exacerbated loss of RBC deformability (Fig. 6d). This was found to lead to marked activation of the Lu/BCAM and CD44 adhesion molecules (Fig. 6e).
Lastly, next to enhanced degradation of otherwise healthy RBC, AI is also characterized by reduced erythropoiesis. We found that erythroblasts, in contrast to erythrocytes, bind SDF-1 (Fig. 6f).

Taken together, these data indicate that healthy RBCs are targeted for destruction upon binding of IL-8 to DARC, thereby contributing to the rapid decrease of circulating RBCs as observed in AI. Furthermore, not only do we show that DARC is capable of simultaneously binding various chemokines, but we also determined that this strongly affects the signalling response in RBCs, with a large impact on their deformability and degradation. We hypothesize that the IL-8-dependent signalling response that is elicited in RBCs similarly occurs in erythroblasts, such that chronic inflammation may substantially impact erythropoiesis through DARC-mediated signalling (Figure 7). In summary, we hypothesize that DARC-mediated signalling contributes to increased breakdown of RBC as well as to the inhibition of erythropoiesis in AI.

Next, the effect of an inhibitor of the Gardos channel in response to pro-inflammatory chemokines was assessed. As demonstrated by the present inventors (Klei *et al* 2020, accepted for publication), aged erythrocytes and erythrocytes that are otherwise prone to clearance from the circulation show an increased adhesion to laminin-α5 (the ligand of Lu/BCAM), and hyaluronic acid (ligand of CD44) on the surface of the RBC, in response to Ca²⁺ influx induced dehydration (Gardos effect). This interaction between adhesion molecules and laminin-α5 (the ligand of Lu/BCAM) and/or hyaluronic acid is thus assessed as a measure of RBC dehydration.

Therefore, erythrocyte adhesion to laminin-α5 and hyaluronic acid in response to IL-8 and sera of sepsis patients was assessed in the absence and presence of TRAM34, an inhibitor of the Gardos channel.

As demonstrated in figure 8A, adhesion of donor RBCs is enhanced by IL-8 incubation. Adhesion of donor RBCs in response to IL-8 incubation is inhibited by the inhibitor of the Gardos channel, TRAM34 (figure 8B) and by anti-DARC antibodies (figure 8C). Importantly, adhesion of donor RBCs in response to sera of sepsis patients is also inhibited by the inhibitor of the Gardos channel, TRAM34 and anti-DARC antibodies (figure 8D).

### Example 2

In this example, the use of a mouse model in studying the efficacy of Senicapoc, an inhibitor of the RBC Gardos channel, on anaemia of inflammation (AI) is described. In the mouse model AI can be induced through injection of heat-killed *B. abortus* (BA). This model resembles AI in humans closely, RBC degradation was indeed found to be a resultant of increased apoptosis of RBC in circulation. In this protocol the efficacy of Senicapoc to limit AI *in* vivo Senicapoc, an inhibitor of the RBC Gardos channel, is investigated. Senicapoc is expected to limit the development of anaemia during systemic inflammation. *In vitro,* we have obtained strong evidence that Senicapoc counteracts Al-mediated degradation of RBC (see Example 1). Senicapoc has been shown to be well-tolerated. In summary, the results from this Example will illustrate the use of Senicapoc as a potential treatment for anaemia during systemic inflammation.

In short, mice have been injected i.p. with heat-killed BA, after which control mice developed anaemia over a 14 day time course. The experimental group has also been injected with heat-killed BA, however, they have been treated with Senicapoc over the course of the experiment. 6 animals have been used for each condition. For animals uninfected who received only carrier, 3 mice were used per experiment, based on the assumption that these mice will not show great variation within the group.

We used this experimental set up, which aims to compare control and Senicapoc treated mice after BA induced systemic inflammation, to base the power analysis on. We identified the hematocrit of our groups after BA injection to be the primary read-out. Using the hematocrit as read-out, the power analysis is by means in which we anticipate that the difference in hematocrit is at least 10% in the Senicapoc-treated animals. We set the alpha at 0.05 and a power of 90%. The decrease in hematocrit is estimated from Kautz et al. (2014), with a 45% decrease in Ht in WT mice after BA injection. This power analysis resulted in an estimation of a 6 mice per group.

### References

- van den Akker, E., et al. The majority of the in vitro erythroid expansion potential resides in CD34(-) cells, outweighing the contribution of CD34(+) cells and significantly increasing the erythroblast yield from peripheral blood samples. Haematologica 95, 1594-1598, doi:10.3324/haematol.2009.019828 (2010).
- Ataga KI, et al. Efficacy and safety of the Gardos channel blocker, senicapoc (ICA-17043), in patients with sickle cell anemia. Blood 2008;111:3991-7.
- Durpes MC, et al. Effect of interleukin-8 and RANTES on the Gardos channel activity in sickle human red blood cells: role of the Duffy antigen receptor for chemokines. Blood Cells Mol Dis. 2010;44:219-23.
- Heideveld, E. et al. CD14+ cells from peripheral blood positively regulate hematopoietic stem and progenitor cell survival resulting in increased erythroid yield. Haematologica 100, 1396-1406, doi:10.3324/haematol.2015.125492 (2015).
- Klei TRL et al. Differential interaction between DARC and SDF-1 on erythrocytes and their precursors. Sci Rep. 2019 Nov 7;9(1):16245. doi: 10.1038/s41598-019-52186-6.Klei TRL. et al. Hemolysis in the spleen drives erythrocyte turnover. Blood. 2020 Oct 1;136(14):1579-1589. doi: 10.1182/blood.2020005351.
- Klei TRL. et al. The Gardos effect drives erythrocyte senescence and leads to Lu/BCAM and CD44 adhesion molecule activation; 2020 accepted for publication in Blood Advances.
- Leberbauer, C. et al. Different steroids co-regulate long-term expansion versus terminal differentiation in primary human erythroid progenitors. Blood 105, 85-94, doi:10.1182/blood-2004-03-1002 (2005).
- Patterson et al., "Expression of the Duffy Antigen/Receptor for Chemokines (DARC) by the Inflamed Synovial Endothelium," J. Pathol. 197(1): 108-116 (2002)
- Rivera A, et al. Modulation of Gardos channel activity by cytokines in sickle erythrocytes. Blood. 2002;99(1):357-603.
- Wulff and Castle. Therapeutic potential of KCa3.1 blockers: an overview of recent advances, and promising trends. Expert Rev Clin Pharmacol. 2010 May; 3(3): 385-396.
- van Zwieten, R. et al. Partial pyruvate kinase deficiency aggravates the phenotypic expression of band 3 deficiency in a family with hereditary spherocytosis. Am J Hematol 90, E35-39, doi:10.1002/ajh.23899 (2015).

## Claims

1. A compound selected from the group consisting of:
- an inhibitor of the Ca²⁺-activated potassium channel (Gardos channel),
- an inhibitor of interaction of one or more chemokines with Duffy antigen receptor for chemokines (DARC), wherein said inhibitor is an antibody or antigen-binding part thereof that specifically binds to DARC, and
- a compound that inhibits activation of adhesion molecules expressed on erythrocytes, wherein said adhesion molecules are Lu/BCAM and/or CD44,
for use in a method for the treatment or prevention of anaemia of inflammation.

2. Compound for use according to claim 1 wherein the compound is an inhibitor of the Gardos channel.

3. Compound for use according to claim 2 wherein the compound is senicapoc, TRAM-34 or NS6180.

4. Compound for use according to claim 1 wherein the compound is an inhibitor of interaction of one or more chemokines with DARC optionally wherein said chemokine is selected from the group consisting of interleukin 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 and combinations thereof.

5. Compound for use according to claim 1 wherein the compound is a compound that inhibits activation of adhesion molecules expressed on erythrocytes, optionally wherein said inhibitor is an anti-Lu/BCAM and/or anti-CD44 antibody.

6. Compound for use according to any one of claims 1 to 5 wherein the treatment or prevention counteracts erythrocyte dehydration, loss of deformability of erythrocytes and/or activation of adhesion molecules Lu/BCAM and/or CD44 expressed on erythrocytes.

7. Compound for use according to any one of claims 1 to 6 wherein the anaemia is hemolytic anaemia.

8. Compound for use according to any one of claims 1 to 7 wherein the treatment further comprises erythrocyte transfusion.

9. Compound for use according to any one of claims 1 to 8 wherein the treatment or prevention comprises administering said compound to a subject suffering from inflammatory disease, such as bacterial or viral infection, autoimmune diseases, cancer, rejection after organ transplantation or chronic kidney disease.

10. A compound selected from the group consisting of:
- an inhibitor of the Ca²⁺-activated potassium channel (Gardos channel), and
- an inhibitor of interaction of one or more chemokines with Duffy antigen receptor for chemokines (DARC), wherein said inhibitor is an antibody or antigen-binding part thereof that specifically binds to DARC,
for use in a method for reducing or preventing erythrocyte dehydration in a subject suffering from chronic inflammation, wherein the subject is suffering from a disorder selected from the group consisting of a viral, bacterial, parasitic or fungal infection, sepsis, cancer, an auto-immune disorder, rejection after organ transplantation, and chronic kidney disease.

11. Compound for use according to claim 10, wherein said auto-immune disorder is selected from the group consisting of rheumatoid arthritis, systemic lupus erythematosus, vasculitis, sarcoidosis and inflammatory bowel disease.

12. Compound for use according to claim 10 or 11 wherein the compound is a Gardos channel inhibitor.

13. Compound for use according to claim 12 wherein the compound is senicapoc, TRAM-34 or NS6180.

14. Compound for use according to claim 10 or 11 wherein said compound is an inhibitor of binding of a chemokine to Duffy antigen receptor for chemokines (DARC) expressed on erythrocytes, optionally wherein said chemokine is selected from the group consisting of interleukin 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 and combinations thereof.

## Patentansprüche

1. Verbindung, ausgewählt aus der Gruppe bestehend aus:
- einem Inhibitor des Ca²⁺-aktivierten Kaliumkanals (Gardos-Kanal),
- einem Inhibitor der Wechselwirkung eines oder mehrerer Chemokine mit dem Duffy-Antigen-Rezeptor für Chemokine (DARC), wobei der Inhibitor ein Antikörper oder ein antigenbindender Teil davon ist, der spezifisch an DARC bindet, und
- einer Verbindung, welche die Aktivierung von auf Erythrozyten exprimierten Adhäsionsmolekülen inhibiert, wobei die Adhäsionsmoleküle Lu/BCAM und/oder CD44 sind,
zur Verwendung in einem Verfahren zur Behandlung oder Prävention von Anämie bei Entzündung.

2. Verbindung zur Verwendung nach Anspruch 1, wobei die Verbindung ein Inhibitor des Gardos-Kanals ist.

3. Verbindung zur Verwendung nach Anspruch 2, wobei die Verbindung Senicapoc, TRAM-34 oder NS6180 ist.

4. Verbindung zur Verwendung nach Anspruch 1, wobei die Verbindung ein Inhibitor der Wechselwirkung eines oder mehrerer Chemokine mit DARC ist, wobei optional das Chemokin ausgewählt ist aus der Gruppe bestehend aus Interleukin 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 und Kombinationen davon.

5. Verbindung zur Verwendung nach Anspruch 1, wobei die Verbindung eine Verbindung ist, welche die Aktivierung von auf Erythrozyten exprimierten Adhäsionsmolekülen inhibiert, wobei optional der Inhibitor
ein Anti-Lu/BCAM und/oder Anti-CD44 Antikörper ist.

6. Verbindung zur Verwendung nach einem der Ansprüche 1 bis 5, wobei die Behandlung oder Prävention der Dehydrierung der Erythrozyten, dem Verlust der Verformbarkeit der Erythrozyten und/oder der Aktivierung der auf den Erythrozyten exprimierten Adhäsionsmoleküle Lu/BCAM und/oder CD44 entgegenwirkt.

7. Verbindung zur Verwendung nach einem der Ansprüche 1 bis 6, wobei die Anämie eine hämolytische Anämie ist.

8. Verbindung zur Verwendung nach einem der Ansprüche 1 bis 7, wobei die Behandlung ferner eine Erythrozytentransfusion umfasst.

9. Verbindung zur Verwendung nach einem der Ansprüche 1 bis 8, wobei die Behandlung oder Prävention Verabreichen der Verbindung an einen Patienten umfasst, der an einer entzündlichen Erkrankung, wie z. B. einer bakteriellen oder viralen Infektion, Autoimmunerkrankungen, Krebs, Abstoßung nach Organtransplantation oder chronischer Nierenerkrankung leidet.

10. Verbindung, ausgewählt aus der Gruppe bestehend aus:
- einem Inhibitor des Ca²⁺-aktivierten Kaliumkanals (Gardos-Kanal), und
- einem Inhibitor der Wechselwirkung eines oder mehrerer Chemokine mit dem Duffy-Antigen-Rezeptor für Chemokine (DARC), wobei der Inhibitor ein Antikörper oder ein antigenbindender Teil davon ist, der spezifisch an DARC bindet,
zur Verwendung in einem Verfahren zur Verringerung oder Prävention von Erythrozytendehydratation bei einem Patienten, der an einer chronischen Entzündung leidet, wobei der Patient an einer Erkrankung leidet, ausgewählt aus der Gruppe bestehend aus einer viralen, bakteriellen,
parasitären oder Pilzinfektion, Sepsis, Krebs, einer Autoimmunerkrankung, Abstoßung nach Organtransplantation und chronischer Nierenerkrankung

11. Verbindung zur Verwendung nach Anspruch 10, wobei die Autoimmunerkrankung ausgewählt ist aus der Gruppe bestehend aus rheumatoider Arthritis, Systemlupus erythematodes, Vaskulitis, Sarkoidose und entzündlicher Darmerkrankung.

12. Verbindung zur Verwendung nach Anspruch 10 oder 11, wobei die Verbindung ein Gardos-Kanalinhibitor ist.

13. Verbindung zur Verwendung nach Anspruch 12, wobei die Verbindung Senicapoc, TRAM-34 oder NS6180 ist.

14. Verbindung zur Verwendung nach Anspruch 10 oder 11, wobei die Verbindung ein Inhibitor der Bindung eines Chemokins an den Duffy-Antigen-Rezeptor für Chemokine (DARC) ist, der auf Erythrozyten exprimiert wird, wobei optional das Chemokin ausgewählt ist aus der Gruppe bestehend aus Interleukin 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 und Kombinationen davon.

## Revendications

1. Composé sélectionné dans le groupe consistant en :
- un inhibiteur du canal potassique activé par le Ca²⁺ (canal Gardos),
- un inhibiteur de l'interaction d'une ou de plusieurs chimiokines avec le récepteur d'antigène Duffy pour les chimiokines (DARC), dans lequel ledit inhibiteur est un anticorps ou une partie de celui-ci se liant à l'antigène qui se lie spécifiquement à DARC, et
- un composé qui inhibe l'activation de molécules d'adhésion exprimées sur les érythrocytes, dans lequel lesdites molécules d'adhésion sont Lu/BCAM et/ou CD44,
destiné à être utilisé dans un procédé de traitement ou de prévention de l'anémie inflammatoire.

2. Composé destiné à être utilisé selon la revendication 1, dans lequel le composé est un inhibiteur du canal Gardos.

3. Composé destiné à être utilisé selon la revendication 2, dans lequel le composé est le sénicapoc, TRAM-34 ou NS6180.

4. Composé destiné à être utilisé selon la revendication 1, dans lequel le composé est un inhibiteur de l'interaction d'une ou de plusieurs chimiokines avec DARC, facultativement dans lequel ladite chimiokine est sélectionnée dans le groupe consistant en l'interleukine 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 et des combinaisons de celles-ci.

5. Composé destiné à être utilisé selon la revendication 1, dans lequel le composé est un composé qui inhibe l'activation de molécules d'adhésion exprimées sur les érythrocytes, facultativement dans lequel ledit inhibiteur est un anticorps anti-Lu/BCAM et/ou anti-CD44.

6. Composé destiné à être utilisé selon l'une quelconque des revendications 1 à 5, dans lequel le traitement ou la prévention neutralise la déshydratation des érythrocytes, la perte de déformabilité des érythrocytes et/ou l'activation des molécules d'adhésion Lu/BCAM et/ou CD44 exprimées sur les érythrocytes.

7. Composé destiné à être utilisé selon l'une quelconque des revendications 1 à 6, dans lequel l'anémie est l'anémie hémolytique.

8. Composé destiné à être utilisé selon l'une quelconque des revendications 1 à 7, dans lequel le traitement comprend en outre une transfusion d'érythrocytes.

9. Composé destiné à être utilisé selon l'une quelconque des revendications 1 à 8, dans lequel le traitement ou la prévention comprend l'administration dudit composé à un sujet souffrant d'une maladie inflammatoire, comme une infection bactérienne ou virale, des maladies auto-immunes, le cancer, un rejet après une greffe d'organe ou la maladie rénale chronique.

10. Composé sélectionné dans le groupe consistant en :
- un inhibiteur du canal potassique activé par le Ca²⁺ (canal Gardos), et
- un inhibiteur de l'interaction d'une ou de plusieurs chimiokines avec le récepteur d'antigène Duffy pour les chimiokines (DARC), dans lequel ledit inhibiteur est un anticorps ou une partie de celui-ci se liant à l'antigène qui se lie spécifiquement à DARC,
destiné à être utilisé dans un procédé de réduction ou de prévention de la déshydratation des érythrocytes chez un sujet souffrant d'une inflammation chronique, dans lequel le sujet souffre d'un trouble sélectionné dans le groupe consistant en une infection virale, bactérienne, parasitaire ou fongique, un sepsis, un cancer, un trouble auto-immun, un rejet après une greffe d'organe, et la maladie rénale chronique.

11. Composé destiné à être utilisé selon la revendication 10, dans lequel ledit trouble auto-immun est sélectionné dans le groupe consistant en la polyarthrite rhumatoïde, le lupus érythémateux systémique, la vascularite, la sarcoïdose et une maladie intestinale inflammatoire.

12. Composé destiné à être utilisé selon la revendication 10 ou 11, dans lequel le composé est un inhibiteur du canal Gardos.

13. Composé destiné à être utilisé selon la revendication 12, dans lequel le composé est le sénicapoc, TRAM-34 ou NS6180.

14. Composé destiné à être utilisé selon la revendication 10 ou 11, dans lequel ledit composé est un inhibiteur de la liaison d'une chimiokine à un récepteur d'antigène Duffy pour les chimiokines (DARC) exprimé sur les érythrocytes, facultativement dans lequel ladite chimiokine est sélectionnée dans le groupe consistant en l'interleukine 8 (IL-8, CXCL8), RANTES (CCL5), CXCL5, CXCL6, CXCL11, CCL17, CXCL1, CXCL2, CXCL3, CXCL4, CCL7, CCL11, CCL13, CCL14, CCL2, CCL1, CCL8, CCL16, CCL18, CXCL9, CXCL10, CXCL13, CXCL12 et des combinaisons de celles-ci.
